(19) Europäisches Patentamt
European Patent Office
Office européen des brevets

(11) Publication number: 0 119 932
B1

(12) **EUROPEAN PATENT SPECIFICATION**

(45) Date of publication of patent specification: **13.05.87**

(51) Int. Cl.$^4$: **C 07 C 101/447,**
C 07 C 99/00, A 61 K 31/215

(21) Application number: **84400549.6**

(22) Date of filing: **19.03.84**

(54) **New 2-((2,6-dichlorophenyl)amine)phenylacetoxyacetyl derivatives, the process for preparing the same and their use in therapeutics.**

(30) Priority: **21.03.83 ES 520813**

(43) Date of publication of application:
**26.09.84 Bulletin 84/39**

(45) Publication of the grant of the patent:
**13.05.87 Bulletin 87/20**

(84) Designated Contracting States:
**AT BE CH DE FR GB IT LI LU NL SE**

(56) References cited:
**DE-A-1 618 465**
**FR-A-1 604 832**

(73) Proprietor: **PRODES S.A.**
**rue Trabajo s/n.**
**San Justo Desvern (Barcelona) (ES)**

(72) Inventor: **Casas, Antonio Vila**
**Rue du Dr. Roux 59-61**
**Barcelone (ES)**

(74) Representative: **Chauchard, Robert et al**
**c/o Cabinet Malémont**
**42, avenue du Président Wilson**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to the 2-[(2,6-dichlorophenyl)amine]phenylacetoxyacetyl derivatives of medical use having the following formula:

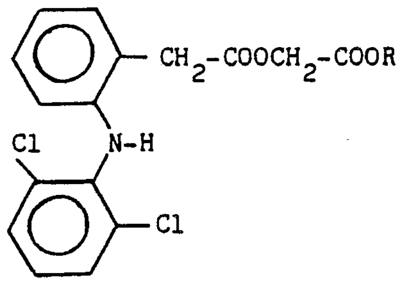

( I )

wherein R can be hydrogen or benzyl, as well as monobasic salts of pharmaceutically acceptable organic or inorganic cations thereof, if R means hydrogen.

The compounds of the present invention can be prepared by means of a process which consists in reacting a 2-[(2,6-dichlorophenyl)amine]phenylacetic acid salt of formula:

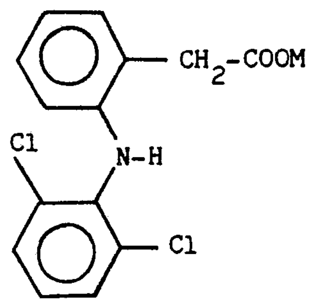

( II )

wherein M represents an alkaline metallic cation (preferably sodium or potassium) with a haloacetate of benzyl III (normally bromoacetate)

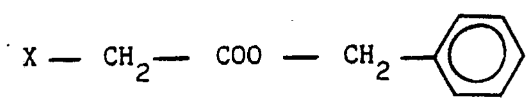

( III )

wherein X is an halogen atom, by heating to 50—150°C in an anhydrous organic medium of polar and aprotic character (for instance dimethylformamide, acetonitrile, dimethylsulphoxide, etc . . . ), during a period which oscillates of from 2 to 20 hours.

In the case where R is an hydrogen, preparation is completed by hydrogenation of the benzyl ester, formula I, obtained by the former process, at a temperature of 25—70°C and pressure of 1—6 atmospheres, by means of the catalytic action of a transition metal (preferably Pd absorbed on carbon).

The new 2-[(2,6-dichlorophenyl)amine]phenylacetoxyacetyl derivatives, according to the present invention, are compounds of therapeutical use with anti-inflammatory and analgesic pharmacologic properties, as shown by experimental pharmacologic data, suitable in the treatment of numerous clinic troubles with inflammation and/or ache, such as rheumatoid, arthritis, ankylosing spondylitis, lumbar pains, sciatica, traumatisms, luxations, etc . . .

As examples of most similar compounds of the prior art, there can be cited the ones described in DE—A—1618465 [alkyl esters of φ-(2,6-dichloroanilino)phenylacetic acid or in FR—A—1604832 [salicylic acid esters of {(dichloro-2,6-anilino)-2}phenylacetic acid], said prior art compounds showing anti-inflammatory and analgesic properties.

The analgesic activity of I(R=H) has been determined by three classical methods:

a) *Test of abdominal contractions by acetic acid*

This test is realized as to the experimental model described by Koster R., Anderson, M. and De Bear, E. J. Fed. Proc., 18, 412 (1959), consisting in administering to a lot of male mice through a gastric catheter, a suspension of the product to be tested half an hour before injecting intraperitoneally 0.2 ml of a 2% acetic acid solution to the mice. There are then counted the number of abdominal contractions, manifested by drawing the rear third of the animal as a consequence of the chemical aggression incited by the acetic acid.

The compound has been tested at two dosages with the results as shown in Table 1.

TABLE 1

| No. animals | Dosage | % protection with respect to control |
|---|---|---|
| 16 | 100 mg/kg | 30.81% |
| 16 | 250 mg/kg | 67.62% |

b) *Test of hot plate thermic stimulus in mouse*

This test is carried through as to the experimental model of Wolfe, G. and MacDonal, A. D., described in (3. Pharm. Exper. Therrap. 80, 300 (1944).

This test consists in administering by a gastric catheter a suspension of the compound at the indicated dosages, and after 30', 1 h., 1.30 h. and 2 h. determinations are being done. Animals are placed onto the hot plate at the temperature of 56°C.

Table 2 indicates the percentage of protection of the dosages of active principle in comparison with a group of control mice.

TABLE 2

| No. animals | Dosage | % protection |
|---|---|---|
| 10 | 20 mg/kg | 22.44% |
| 10 | 40 mg/kg | 24.80% |

c) *Podolororimetric test in a rat*

This test is based upon the technique described by Randall L. and Selitto, 3. (Arch. Int. Pharmacodyn. 111, 409 (1957), consisting in administering the drug in suspension in a 1% Carboxymethylcellulose (C.M.C.).

After 30 minutes there is administered a 20% yeast suspension into the left leg into the right leg the same amount of physiologic serum. There is determined the threshold of the supported weight by both legs after 1 h., 2 h. and 4 h. after the administration of the yeast.

Table 3 states the percentage of protection of the dosage of active principle in comparison with a group of control rats.

TABLE 3

| No. animals | Dosage | % protection |
|---|---|---|
| 10 | 2.5 mg/kg | 32.05% |
| 10 | 10 mg/kg | 45.45% |

The anti-inflammatory activity of the two compounds has been studied by means of the test of the plantar edema of carrageenin (C. A. Winter, Proc. Soc. Exp. Brol. Med. 111, 544—47 (1962).

The method consists in preparing homogeneous lots of albino rats Sprague-Dawley, males, with weights of from 180—200 g to which is being administered by gastric catheter the compound suspended in a vehicle constituted by C.M.C. 0.2% and Twenn 80 0.1%, 60 (sixty) minutes before the production of the subplantar oedema. The administered amount was 10 mg/kg in all cases, the control lot receiving the same amount of vehicle. Immediately after this, all animals receive via oral administration an amount of 20 ml/kg of distilled water to attain an uniform hydration. 60 minutes later there is administered via subcutaneous into the left rear leg a 1% solution of carrageenin physiologic serum, at the dosage of 0.1 ml/rat. The same amount of physiologic serum is then injected into the right rear part.

The plantar amount of both rear extremities is determined before, and 1, 2, 3, 4, 5 and 6 hours after the administration of the carrageenin solution, by means of a mercuryplethysmograph Ugo Basile, model 7.100.

The sample drug as used was an Indometacine. There is calculated the percentage representing the value of each area with respect to the value of the area of the control group to which is assigned the value of 100% of inflammation. And running from these data there is then calculated its complementary value, thus obtaining the percentage of inhibition.

There is then determined the arithmetic average and standard deviation ($M\pm\varphi$) of the obtained values as to the volumes of each leg, normal or inflamed, for each animal lot (control and treated lot).

Table 4 shows the results found when administering the products (IR=H; IR=$CH_2$—$C_6H_5$).

TABLE 4

| Product | Dosage | No. animals | *% area relative* Inflammation (M ± φ) | Inhibition % (M ± φ) |
|---|---|---|---|---|
| CONTROL | 10 ml/kg | 40 | 100 | 0 |
| INDOMETACINE | 2.5 mg/kg | 40 | 53.48 ± 7.82 | 46.52 |
| I, R=H | 3.0 mg/kg | 32 | 63.25 ± 5.88 | 33.75 |
| I, R=$CH_2$—$C_6H_5$ | 3.75 mg/kg | 32 | 64.04 ± 7.91 | 35.96 |

*The acute toxicity* of these compounds has been studied orally in rats. There has been determined the $LD_0$ and $LD_{50}$, and the limits of reliance by the technique of Litchfield and Wilcoxon. Table 5 below summarizes the results.

TABLE 5

| Compound | $LD_{50}$ (mg/kg) | $LD_0$ (mg/kg) |
|---|---|---|
| I, R=H | 154.06<br>120.92<br>94.91 | 68.03 |
| I, R=$CH_2$—$C_6H_5$ | 130.16<br>95.31<br>69.77 | 38.51 |

*The subacute toxicity* of both compounds has been studied (IR=H, IR=$CH_2C_6H_5$) after oral administration, by catheter, during one month in Wistar rats of both sex with 120 animals per lot. The administration has been realized 6 days per week. The used dosages have been 15 mg/kg/day, 50 mg/kg/day and 100 mg/kg/day. A lot of control animals only received excipient (C.M.C. 1% suspension). The parameters which have been studied are: the evolution of mortality, weight rates, diet consumption, hematic, biochemical and urinary parameters, and the histopathologic study showed that only at a dosage of 100 mg/kg/day (high dosage) and for I, R=H, there was some case of mortality with a manifestation of blood in the excrements and the histologic study showed the presence of irritation of the gastric and/or intestinal of the mucous membrane at the stated high dosage.

*The fetal and teratogenous toxicity study* of the compounds (IR=H, IR=$CH_2$—$C_6H_5$) in Sprague-Dawley rats and fetus of first generation, and using a dosage of 15 mg/kg/day, 50 mg/kg/day and 75 mg/kg/day of the product via oral administration and during the period of organogenesis has shown that the product subject of the present invention does not produce any toxic effects to the gestating females and its breedings and neither induces teratogenous effects.

The compounds subject of the present invention can be used as drugs in human therapy. The daily dosage via oral administration is in the range of from 50 to 150 mg. for I, R=H, and of from 150 to 200 mg for I, R=$CH_2C_6H_5$. It can be administered under the form of a pharmaceutical composition in combination with a pharmaceutically acceptable carrier or vehicle, for example under the form of tablets, recovered tablets, capsules, syrup and suppositories. In the case of I, R=H the soluble salts (for instance sodic) can be administered under an injectable form.

Hereunder are some examples of pharmaceutical ways of administering these products:

| *Tablets* | I, R=H | I, $R=CH_2—C_6H_5$ |
|---|---|---|
| Compound | 55.6 | 63.6 |
| Lactose | 50.0 | 42 |
| Ac.di.sol | 10.0 | 10.0 |
| Magnesium stearate | 2.4 | 2.4 |

| *Suspension* | I, R=H | I, $R=CH_2—C_6H_5$ |
|---|---|---|
| Compound | 166.8 | 180.0 |
| Aerosil P—101 | 100.0 | 100.0 |
| Sorbitol 70% | 2,000 | 2,000 |
| Methylparaben | 7.5 | 7.5 |
| Propylparaben | 2.5 | 2.5 |
| Water c.s.p. | 150.0 | 136.8 |

| *Suppositories* | I, R=H | I, $R=CH_2—C_6H_5$ |
|---|---|---|
| Compound | 75 mg. | 100 mg. |
| Stearinum mass C | 2.5 g. | 2.5 g. |

To facilitate the disclosure, following examples comprise the way of realization only for illustrative purposes and never limiting the scope of the invention.

Example 1

*Benzyl 2-[(2,6-dichlorophenyl)amine]phenylacetoxyacetate*

50 g (0.166 mole) of sodic 2-[(2,6-dichlorophenyl)amine]phenylacetate are solved in 300 ml of N,N'-dimethylformamide, under heating to 50°C and 44.220 g (0.193 mole) of benzyl bromoacetate are added. The mixture is stirred under these conditions during 8 hours. Then the reaction solvent is eliminated at reduced pressure and sodic salts are precipitated by adding 400 ml of ethyl ether. The ethereous phase is then filtered and washed with 3×100 ml of water and dried on sodic sulphate. Finally, it is concentrated until obtaining an oil which is washed twice with 100 ml of hexane and it is crystallized from the hexane/ether mixture (depreciating the first darker fraction separated in the form of oil). There are obtained 44.1 g (70%) of 2-[(2,6-dichlorophenyl)amine]phenylacetoxyacetate of benzyl, in the form of white crystals whose melting point is 67—69°C. Rf=0.73 (acetone 10/chloroform 90).

IR: (KBr)$\nu_{max}$ 3370, 3000, 1755, 1735, 1570, 1500, 1440, 1420, 1390, 1360, 1290, 1270, 1190, 1140, 770, 750 and 700 $cm^{-1}$.

$H^1$ NMR: ($CDCl_3$) ppm 6.5—7.4 (m, 12H, aromatic), 5.15 (s, 2H, $OC\underline{H}_2$-Ar), 4.7 (s, 2H; O—$C\underline{H}_2$—COO), 3.9 (s, 2H, Br—$C\underline{H}_2$—COO)

*Elemental analysis*

Empiric formula $C_{23}H_{19}Cl_2NO_4$

| | C% | H% | Cl% | N% |
|---|---|---|---|---|
| Calculated | 62.17 | 4.31 | 15.95 | 3.15 |
| Found | 61.99 | 4.33 | 15.98 | 3.13 |

*2-[(2,6-dichlorophenyl)amine]phenylacetoxyacetic acid*

32 g (0.072 mole) of benzyl 2-[(2,6-dichlorophenyl)amine]phenylacetoxyacetate are solved in 1000 ml

of acetic acid; the solution is mixed with 5 g of Pd/C 10% and then hydrogenated at a pressure of 3 atmospheres, under heating to 40°C during 10 hours. The catalyst is separated by filtration, the solution is concentrated and the resulting solution is then washed with toluene to eliminate the rest of acetic acid. Finally, it is recrystallized from cyclohexane thus obtaining 13 g (50%) of 2-[(2,6-dichlorophenyl)amine]phenylacetoxyacetic acid, white crystals whose melting point is 149—50°C. Rf=0.51 (acetone 100/acetic acid 2.5).

IR: (KBr) $\nu_{max}$: 3320 (env), 1770, 1715, 1580, 1570, 1490, 1440, 1415, 1340, 1280, 1250, 1130 (env), 1050, 1050, 960, 930, 900, 850, 770, 750, and 710 $cm^{-1}$.
$H^1$ NMR: ($CDCl_3$), ppm 8.6—8.9 (s, 1H, COO<u>H</u>), 6.3—7.5 (m, 8H, aromatics and N—H), 4.65 (s, 2H, $COOC\underline{H_2}$—COO), 3.9 (s, 2H, Ar—$C\underline{H_2}0$—COO).
UV spectrum λ max=275 nm. Log ε=4.14 (EtOH).

Elemental analysis
Empiric formula $C_{16}H_{13}Cl_2NO_4$

| | C% | H% | Cl% | N% |
|---|---|---|---|---|
| Calculated | 54.28 | 3.69 | 20.00 | 3.95 |
| Found | 54.11 | 3.72 | 20.02 | 3.97 |

Example 2

*Benzyl 2-[(2,6-dichlorophenyl)amine]phenylacetoxyacetate*

50 g (0.166 mole) of sodic 2-[(2,6-dichlorophenyl)amine]phenylacetate are solved in 200 ml of N,N'-dimethylformamide, under heating to 50°C. 41.620 g (0.181 mole) of benzyl bromoacetate are added. Under these conditions, the mixture is maintained under stirring during three hours. Once finished the reaction, solvent is eliminated at reduced pressure and sodic salts are precipitated by adding 400 ml of ether. The solution is then filtered, the ethereous phase is washed three times with 100 ml of water and dried on sodic sulphate. It is finally concentrated until obtaining an oil which is washed twice with 100 ml of hexane and crystallized from methanol, thus obtaining 45.280 g (61%) of 2-[(2,6-dichlorophenyl)amine]phenylacetoxyacetate of benzyl.

*2-[(2,6-dichlorophenyl)amine]phenylacetoxyacetic acid*

45.280 g (0.102 mole) of 2-[(2,6-dichlorophenyl)amine]phenylacetoxyacetate of benzyl are solved in 1,500 ml of ethyl acetate; the solution is mixed with 7 g of Pd/C 10% and hydrogenated at atmospheric pressure during 14 hours. The solution is then filtered, concentrated and the residue is crystallized in hexane-ethyl acetate, thus obtaining 23.510 g (65%) of 2-[(2,6-dichlorophenyl)amine]phenylacetoxyacetic acid.

**Claims for the Contracting States: BE CH DE FR GB IT LI LU NL SE**

1. 2-[(2,6-dichlorophenyl)amine]phenylacetoxyacetyl derivatives of formula:

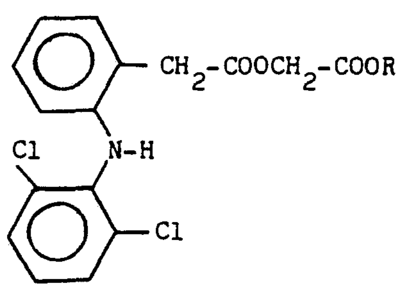

( I )

wherein R can be hydrogen or benzyl, as well as monobasic salts of therapeutically acceptable organic and inorganic cations thereof, when R means hydrogen.

2. 2-[(2,6-dichlorophenyl)amine]phenylacetoxyacetate of benzyl.

3. 2-[(2,6-dichlorophenyl)amine]phenylacetoxyacetic acid.

4. Drug, characterized in that it is constituted by a derivative according to claim 1, 2 or 3.

5. Pharmaceutical composition, characterized in that it comprises as an active component an effective amount of 2-[(2,6-dichlorophenyl)amine]phenylacetoxyacetyl derivatives according to claim 1, 2 or 3 and a pharmaceutically acceptable carrier or vehicle.

6. A process of preparing 2-[(2,6-dichlorophenyl)amine]phenylacetoxyacetyl derivatives according to

claim 1 which consists in reacting a 2-[(2,6-dichlorophenyl)amine]phenylacetic acid salt having the formula:

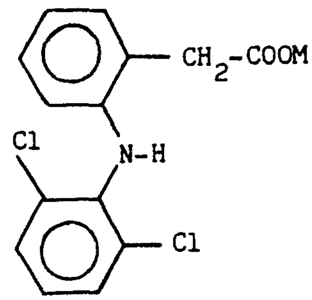

( II )

wherein M represents an alkaline metallic cation, with a benzyl haloacetate of formula:

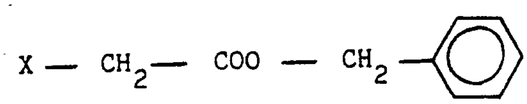

( III )

under heating in an anhydrous organic medium of polar and aprotic character.

7. Process according to claim 6, characterized in that the benzyl haloacetate is the benzyl bromoacetate and M is sodium or potassium.

8. A process of preparing 2-[(2,6-dichlorophenyl)amine]phenylacetoxyacetic acid which consists in subjecting to hydrogenation the 2-[(2,6-dichlorophenyl)amine]phenylacetoxyacetate of benzyl through the catalytic action of a transition metal.

9. Process according to claim 8, characterized in that the transition metal is palladium adsorbed on carbon.

**Claims for the Contracting State: AT**

1. A process for preparing 2-[(2,6-dichlorophenyl)amine]phenylacetoxyacetyl derivatives of formula:

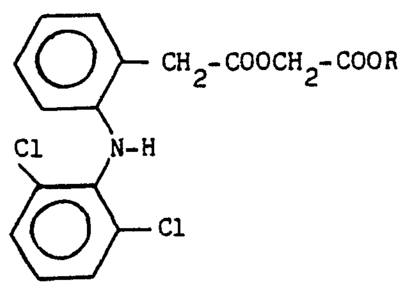

( I )

wherein R can be hydrogen or benzyl, as well as monobasic salts of therapeutically acceptable organic or inorganic cations thereof when R is hydrogen, characterized in that it consists (a) in reacting a 2-[(2,6-dichlorophenyl)amine]phenylacetic acid salt having the formula:

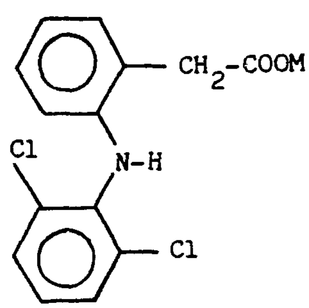

( II )

wherein M represents an alkaline metallic cation, with a benzyl haloacetate of formula:

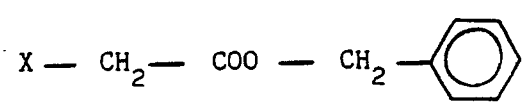

( III )

wherein X is an halogen atom and possibly (b) in subjecting the 2-[(2,6-dichlorophenyl)amine]phenylacetoxyacetate of benzyl thus obtained to hydrogenation in the presence of a transition metal catalyst.

2. Process according to claim 1, characterized in that M is sodium or potassium.

3. Process according to claim 1 or 2, characterized in that reaction (a) is carried out under heating and in an anhydrous organic medium of polar and aprotic character.

4. Process according to claim 1, 2 or 3, characterized in that the benzyl haloacetate is the benzyl bromoacetate.

5. Process according to any one of claims 1 to 4, characterized in that the transition metal catalyst is palladium adsorbed on carbon.

**Patentansprüche für die Vertragsstaaten: BE CH DE FR GB IT LI LU NL SE**

1. 2-[(2,6-Dichlorphenyl)amin]phenylacetoxyessigsäure-Derivate der Formel:

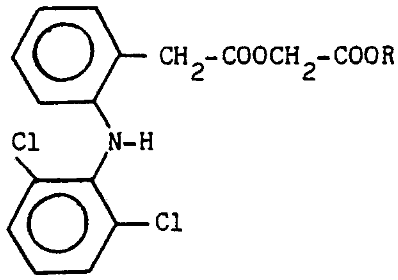

( I )

worin R Wasserstoff oder Benzyl sein kann, sowie monobasische Salze therapeutisch annehmbarer, organischer oder anorganischer Kationen hiervon, wenn R Wasserstoff bedeutet.

2. 2-[(2,6-Dichlorphenyl)amin]phenylacetoxybenzylacetat.

3. 2-[(2,6-Dichlorphenyl)amin]phenylacetoxyessigsäure.

4. Arzneimittel, dadurch gekennzeichnet, dass es aus einem Derivat gemäss Anspruch 1, 2 oder 3 aufgebaut ist.

5. Pharmazeutische Zusammensetzung, dadurch gekennzeichnet, dass sie als Wirkstoff eine wirksame Menge an 2-[(2,6-Dichlorphenyl)amin]phenylacetoxyessigsäure-Derivaten gemäss Anspruch 1, 2 oder 3 und einen pharmazeutisch annehmbaren Träger oder Hilfsstoff umfasst.

6. Verfahren zur Herstellung von 2-[(2,6-Dichlorphenyl)amin]phenylacetoxyessigsäure-Derivaten, bestehend aus der Umsetzung eines 2-[(2,6-Dichlorphenyl)amin]phenylessigsäure-Salzes der Formel:

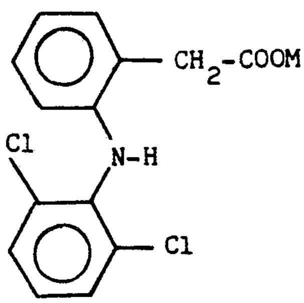

( II )

worin M ein Alkalimetallkation bedeutet, mit einem Benzylhalogenacetat der Formel:

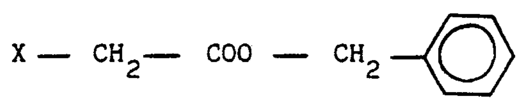

( III )

unter Erwärmen in einem wasserfreien, organischen Medium von polarem und aprotischem Charakter.

7. Verfahren nach Anspruch 6, dadurch gekennzeichnet, dass das Benzylhalogenacetat das Benzylbromacetat und M Natrium oder Kalium ist.

8. Verfahren zur Herstellung von 2-[(2,6-Dichlorphenyl)amin]phenylacetoxyessigsäure, das darin besteht, dass man das 2-[(2,6-Dichlorphenyl)amin]phenylacetoxybenzylacetat durch die katalytische Wirkung eines übergangsmetalls der Hydrierung unterzieht.

9. Verfahren nach Anspruch 8, dadurch gekennzeichnet, dass das übergangsmetall auf Kohlenstoff adsorbiertes Palladium ist.

**Patentansprüche für den Vertragsstaat: AT**

1. Verfahren zur Herstellung von 2-[(2,6-Dichlorphenyl)amin]phenylacetoxyessigsäure-Derivaten der Formel:

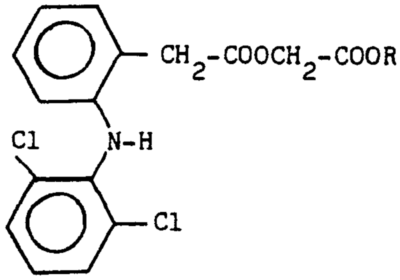

( I )

worin R Wasserstoff oder Benzyl sein kann, sowie monobasische Salze therapeutisch annehmbarer, organischer oder anorganischer Kationen hiervon, wenn R Wasserstoff bedeutet, dadurch gekennzeichnet, dass es besteht aus (a) der Umsetzung eines 2-[(2,6-Dichlorphenyl)amin]phenylessigsäure-Salzes der Formel:

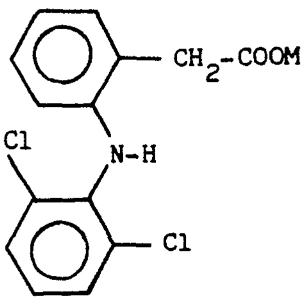

( II )

worin M ein Alkalimetallkation bedeutet, mit einem Benzylhalogenacetat der Formel:

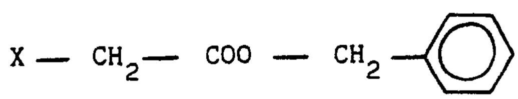

( III )

worin X ein Halogenatom ist und möglicherweise (b) der Unterziehung des so erhaltenen 2-[(2,6-Dichlorphenyl)amin]phenylacetoxybenzylacetats der Hydrierung in Gegenwart eines übergangsmetall-Katalysators.

2. Verfahren nach Anspruch 1, dadurch gekennzeichnet, dass M Natrium oder Kalium ist.

3. Verfahren nach Anspruch 1 oder 2, dadurch gekennzeichnet, dass die Umsetzung (a) unter Erwärmen und in einem wasserfreien, organischen Medium von polaren und aprotischem Charakter durchgeführt wird.

4. Verfahren nach Anspruch 1, 2 oder 3, dadurch gekennzeichnet, dass das Benzylhalogenacetat das Benzylbromacetat ist.

5. Verfahren nach einem der Ansprüche 1 bis 4, dadurch gekennzeichnet, dass der übergangsmetall-Katalysator auf Kohlenstoff adsorbiertes Palladium ist.

**Revendications pour les Etats contractants: BE CH DE FR GB IT LI LU NL SE**

1. Dérivés 2-[(2,6-dichlorophényl)amine]phénylacétoxyacétyle de formule:

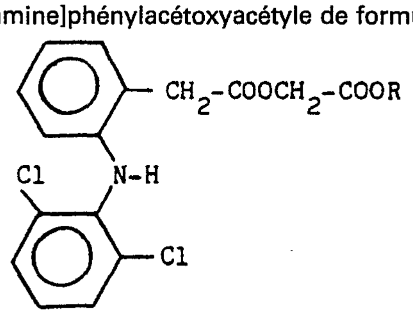

( I )

dans laquelle R peut être hydrogène ou benzyle, ainsi que leurs sels monobasiques à cations minéraux ou organiques, thérapeutiquement acceptables, quand R représente l'hydrogène.

2. 2-[(2,6-dichlorophényl)amine]phénylacétoxyacétate de benzyle.

3. Acide 2-[(2,6-dichlorophényl)amine]phénylacétoxyacétique.

4. Médicament, caractérisé en qu'il est constitué par un dérivé selon la revendication 1, 2 ou 3.

5. Composition pharmaceutique, caractérisée en ce qu'elle comprend, à titre de composant actif, une quantité efficace des dérivés 2-[(2,6-dichlorophényl)amine]phénylacétoxyacétyle selon la revendication 1, 2 ou 3 et un véhicule ou support pharmaceutiquement acceptable.

6. Procédé pour préparer les dérivés 2-[(2,6-dichlorophényl)amine]phénylacétoxyacétyle selon la revendication 1, qui consiste à faire réagir un sel d'acide 2-[(2,6-dichlorophényl)amine]phénylacétique de formule:

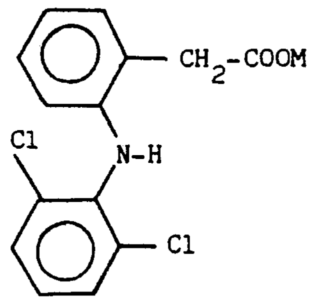

( II )

dans laquelle M représente un cation métallique alcalin, avec un haloacétate de benzyle de formule:

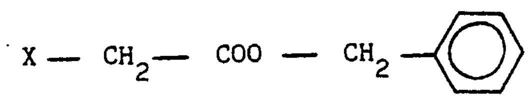

( III )

cette réaction étant effectuée à chaud et dans un milieu organique anhydre à caractère polaire et aprotique.

7. Procédé selon la revendication 6, caractérisé en ce que l'haloacétate de benzyle est le bromoacétate de benzyle et en ce que M est le sodium ou le potassium.

8. Procédé pour préparer l'acide 2-[(2,6-dichlorophényl)amine]phénylacétoxyacétique, qui consiste à soumettre à une hydrogénation le 2-[(2,6-dichlorophényl)amine]phénylacétoxyacétate de benzyle en utilisant l'action catalytique d'un métal de transition.

9. Procédé selon la revendication 8, caractérisé en ce que le métal de transition est du palladium adsorbé sur du carbone.

**Revendications pour l'Etat contractant: AT**

1. Procédé pour préparer les dérivés 2-[(2,6-dichlorophényl)amine]phénylacétoxyacéyle de formule:

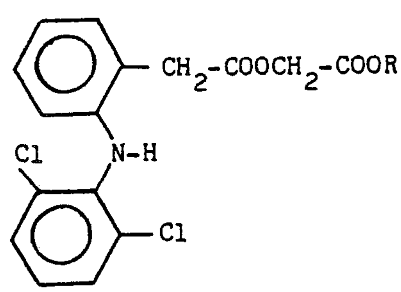

( I )

dans laquelle R peut être hydrogène ou benzyle, ainsi que leurs sels monobasiques à cations minéraux ou organiques, thérapeutiquement acceptables, quand R représente l'hydrogène, caractérisé en ce qu'il consiste (a) à faire réagir un sel d'acide 2-[(2,6-dichlorophényl)amine]phénylacétique, de formule:

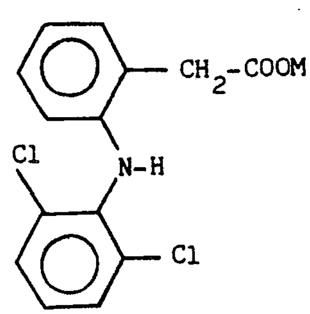

( II )

dans laquelle M représente un cation métallique alcalin, avec un haloacétate de benzyle de formule:

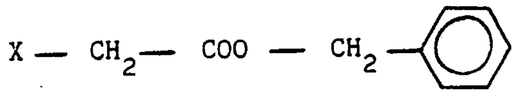

( III )

dans laquelle X est un atome d'halogène et, éventuellement, (b) à soumettre le 2-[(2,6-dichlorophényl)amine]phénylacétoxyacétate de benzyle ainsi obtenu à une hydrogénation en présence d'un catalyseur à base d'un métal de transition.

2. Procédé selon la revendication 1, caractérisé en ce que M est le sodium ou le potassium.

3. Procédé selon la revendication 1 ou 2, caractérisé en ce que la réaction (a) est effectuée à chaud et dans un milieu organique anhydre à caractère polaire et aprotique.

4. Procédé selon la revendication 1, 2 ou 3, caractérisé en ce que l'haloacétate de benzyle est le bromoacétate de benzyle.

5. Procédé selon l'une quelconque des revendications 1 à 4, caractérisé en ce que le catalyseur à base d'un métal de transition est constitué par du palladium adsorbé sur du carbone.